Europäisches Patentamt

European Patent Office

Office européen des brevets

(19)

(11) Publication number: **0 089 122**
**B1**

(12) **EUROPEAN PATENT SPECIFICATION**

(45) Date of publication of patent specification: **13.07.88**

(51) Int. Cl.⁴: **A 61 M 1/18**

(21) Application number: **83300829.5**

(22) Date of filing: **18.02.83**

(54) **Hollow fibre oxygenator, assembly containing same and method for making same.**

(30) Priority: **19.02.82 US 350460**

(43) Date of publication of application:
**21.09.83 Bulletin 83/38**

(45) Publication of the grant of the patent:
**13.07.88 Bulletin 88/28**

(84) Designated Contracting States:
**AT BE CH DE FR GB IT LI NL SE**

(56) References cited:
**EP-B-0 005 866**
**DE-A-2 721 444**
**US-A-3 422 008**
**US-A-3 536 611**
**US-A-3 794 468**
**US-A-3 989 626**
**US-A-4 020 230**
**US-A-4 073 622**
**US-A-4 140 637**
**US-A-4 151 088**
**US-A-4 240 907**

(73) Proprietor: **CD Medical, Inc.**
**3655 N.W. 87th Avenue**
**Miami Florida 33152 (US)**

(72) Inventor: **Mather III, Frank Watkins**
**465 Rideview Drive**
**Pleasant Hill California 94523 (US)**

(74) Representative: **McCall, John Douglas et al**
**W.P. THOMPSON & CO. Coopers Building**
**Church Street**
**Liverpool L1 3AB (GB)**

Courier Press, Leamington Spa, England.

EP 0 089 122 B1

## Description

Extracorporeal oxygenation of blood during cardiac surgery is a problem of long standing and various types of devices have been developed and used for that purpose since the late 1950's. In 1958, DeWall U.S. Patent 2,972,349 proposed thin walled capillary tubes of silicone rubber for use in a device that flowed blood inside the fibre lumens and oxygen outside the fibres in a gas and liquid tight chamber, but the device did not attain commercial acceptance.

Commercial acceptable blood oxygenators up to the present have comprised bubble oxygenators, disc oxygenators, and flat membrane types. Bubble and disc type oxygenator devices typically employ an open blood chamber in which either oxygen is bubbled directly into a pool of blood or rotating discs expose thin films of blood to an oxygen environment as illustrated in U.S. Patent Nos. 3,674,440 and 3,841,837.

Gas permeable sheet membranes mounted in multiple layered plate and frame filter press devices which supply oxygen to the outer surfaces of a thin blood containing envelope of gas permeable material are typified by U.S. Patent 3,332,746. Another sheet membrane device which employs flattened tubular oxygen containing membranes that transfer oxygen and carbon dioxide to blood flowing over the outer membrane surface is shown in U.S. Patent 4,094,792.

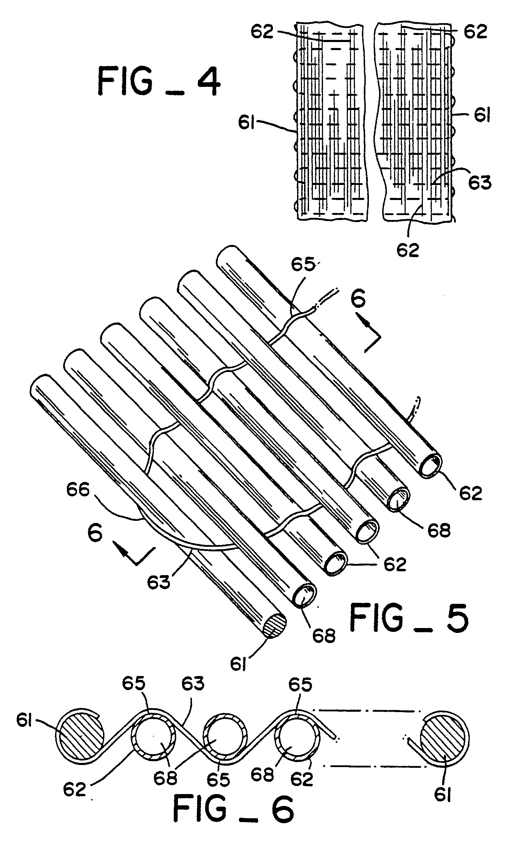

The hollow fibre oxygenator of this invention makes use of hollow fibres that carry oxygen in their lumens and transfer oxygen into and carbon dioxide from blood flowing on the outside of the fibres. Research attempts to use hollow fibres with blood flow inside the fibres and oxygen outside the fibres was conducted by The Dow Chemical Company beginning in 1968 under NHLI Contract PH 43—68—1387 but was abandoned in 1971 in favour of research on the blood-outside hollow fibre concept using fibre winding techniques employed by Dow for hollow fibre reverse osmosis devices. These fibre winding techniques are taught in McLain U.S. Patent 3,422,008 and enable continuous wrapping of fibres on hollow cylindrical supports to form annular mats of fibres. The techniques shown in U.S. Patent 3,422,008 employ a tow of, for example, 16 individual fibres that is laid up as a spiral winding on a cylindrical core as the core rotates with the fibres being looped between spaced apart tubesheets formed by casting resin addition at the ends of the core or at selected spacings along the core. Attempts to use these fibre winding techniques with tows of gas permeable fibres did produce cylindrical devices having the general appearance of the device of Fig. 5 of U.S. Patent 3,422,008.

Certain of the prototype devices did exhibit satisfactory gas transfer characteristics but the good indications could not be consistently repeated due to the lack of control of spacing between individual fibres and non-uniformity of fibre packing density which led to non-uniform flow of blood through the fibre mat. The research was terminated without achieving satisfactory levels of performance relative to commercial blood oxygenators in 1973.

US—A—4240907 describes an apparatus and method for blood dialysis in which toxic substances are removed from the blood by introducing the blood to a dialyser which includes a plurality of layers of semi-permeable tubules wrapped around a central core, said central core including a solid cylindrical section which prevents dialysate flow therethrough.

U.S. 3,536,611 discloses a capillary membrane device which uses a plurality of capillary tubes, the tubes being wrapped around a central perforated distributor tube to provide means for the diffusion of a sweep stream which diffuses radially to the axis of the distributor tube.

US—A—3,794,468 discloses a blood oxygenator element comprising a plurality of layers of hollow tubular conduits, each made of a semi-permeable membrane, stacked on and around a core to form a multilayer winding of the conduit.

An attempt to control fibre spacing in a hollow fibre oxygenator with blood flow on the outside of the fibres was made in a three year research program conducted at the Medical College of Virginia under NHLI Contract No. PH 43—67—1426. A variety of gas permeable hollow fibres made of polyethylene, silicone rubber, methyl pentene polymers, and mixtures of silicone rubber and methyl pentene fibres, as well as mixtures of silicone rubber and polycarbonate fibres were evaluated. Various attempts to weave the hollow fibres into an open weave structure that could be laid up in square sheets, or in annular form supported on a cylindrical core were tried, but the program failed and was terminated. Problems still remaining included insufficient fibre strength, elasticity brittleness, cracking and inability to withstand frictional forces during weaving attempts that result in fibre crushing and flattening.

According to one aspect of the present invention there is provided a blood oxygenator element for the treatment of blood comprising a plurality of hollow gas permeable fibres and a longitudinal hollow blood permeable core, said fibres overlying the peripheral surface of said core in a plurality of contiguous layers, each layer comprising a multitude of said fibres and extending spirally around and along the longitudinal axis of said core, said fibres being spaced laterally from each adjacent fibre in said layer, and said fibres terminating in spaced apart tubesheets joining said fibres to each other and to said core, characterised in that said multitude of fibres in each layer are arranged in a tape with the fibres in each tape extending longitudinally thereof in parallel arrangement distributed across its width, and in that the tapes in alternate layers extend spirally in opposite directions so that the said fibres in each said layer are substantially equally radially distant from said peripheral surface of said core, and that said fibres in each said layer cross the fibres in each radially adjacent layer at an angle of between 30° and 90°.

The tape used in this invention comprises a layer of scores, or hundreds, of substantially parallel, gas permeable hollow fibres in a fixed relationship which provides substanitally uniform lateral spacing between the fibres in the tape. Preferably the tape includes a solid monofilament defining each of the side edges of each tape, and a solid flexible transverse filament for maintaining spacing between said hollow fibres without substantially altering their cross-section at crossover points therewith, said solid flexible transverse filament being woven over and under the adjacent parallel hollow fibres and around the exterior of said monofilament side edges in a continuous traversing pattern along the length of said tape. The transverse filament maintains the desired lateral hollow fibre spacing without crushing or flattening the fibres at crossover points.

The invention further includes a method of making a blood oxygenator element in accordance with the invention which method comprises:

1. providing a core,

2. providing a tape of hollow gas permeable fibres, said tape comprising a plurality of said fibres arranged in substantially parallel spaced apart and substantially planar relation,

3. spirally wrapping said tape on said core to form a plurality of layers of said fibres overlying the peripheral surface of said core, the width of said tape and the diameter of said core being selected such that the edge surface of each successive wrap of said tape in said layer is adjacent to the edge surface of the prior wrap so as to substantially maintain said substantially parallel spaced apart relation between said fibres in each said tape, and covering the peripheral surface of said core for the length of said core, and

4. securing the axially spaced apart ends of said fibres in each layer to each other and to said core in tubesheets having co-planar outer and surfaces in which the open lumens of each said fibre terminate, characterised in that

5. said wrapping to form overlying layers covering the prior formed said layer is achieved by reversing the direction of said spiral such that each tape wrap positions each said fibre in said overlying layer relative to the adjacent fibre in the underlying layer at an angle between about 30° and about 90°, and

6. said core is a hollow blood permeable core.

The new blood oxygenator element of this invention comprises a hollow core member fabricated from any blood compatible material such as polypropylene. The core is preferbaly cylindrical and has a blood permeable wall that serves to support an annular mat of gas permeable, hollow fibre layers laid down upon the peripheral surface of the core member. Each layer of hollow fibres on the peripheral surface of the hollow core member is formed by using the above described new tape of this invention. The tape is helically, or spirally wound on the core such that the side edges of contiguous tapes are immediately adjacent each other to form a continuous layer of hollow gas permeable fibres extending for the full length of the core.

Each layer in the oxygenator element comprises hundreds, or thousands, of hollow fibres that are substantially uniformly spaced from each other, relative to the axis of the core, in a fixed spaced relationship that is substantially maintained during use by virtue of the substantially inelastic nature of the fibres, and the restraining effect of the fine cross thread in the tape, or its equivalent. The successive, contiguous layers of fibres on the core are laid up so that each hollow fibre in each layer is radially equidistant from the peripheral surface of the core. By reversing the angle of spiral, relative to the axis of the core, on each successive layer as the core rotates and tape is applied, the cross over angle at the points of cross over of the individual fibres being applied with the underlying fibres is variably controllable within the range of about 30° and about 90°, and preferably exceeds about 60°. The preferred fibres are microporous polyethylene. After lay up on the core of the desired number of layers of hollow fibres, a resin tubesheet is formed at each end of the core by centrifugal, or dunk potting, and the tubesheets are transversely cut to expose the open lumens of each hollow fibre in the outer end surface of the spaced apart tubesheets.

Successive layers of fibres, having a cross over angle above 30° with the contiguous fibres in the overlying and underlying adjacent layers, form a series of substantially uniformly spaced gaps between layers that interconnect in a substantially radial line. These gaps are small and cause the radially outwardly flowing blood to bathe the outer surface of each fibre with a thin film of blood which is gentle and non-harmful to the blood while concurrently causing an efficient gas exchange through the gas permeable walls of each fibre.

The method of this invention comprises the steps necessary to form the above described new tape of hollow gas permeable fibres, and the steps of winding that tape on a hollow supporting core member to thereby form the new oxygenator element above described.

The new oxygenator element, when incorporated into a cylindrical shell by centrifugally casting a resin tubesheet around the hollow fibres at each end of the core to thereby seal the fibres to each other, to the core and to the inner shell wall, forms the new blood oxygenator device of this invention.

The assembly of this invention comprises a blood reservoir, a blood heater and the new blood oxygenator device of this invention integrated into a single compact, self-supporting, easily handleable unit.

Preferred embodiments of the invention will now be described in detail with reference to the accompanying drawings in which

Fig. 1 is a side elevation view of the blood oxygenator device in the preferred orientation during use;

Fig. 2 is a vertical cross-section of the device of Fig. 1 showing the blood oxygenator element integrated into a preferred form of device for use in oxygenating blood;

Fig. 3 is a cross sectional view of the device shown in Fig. 2 taken along the line 3—3 thereof;

Fig. 4 is a broken away portion of a schematic view of the preferred woven form of the tape of this invention;

Fig. 5 is an enlarged perspective view of the lower left hand corner portion of the tape of Fig. 4;

Fig. 6 is a cross sectional view taken along the line 6—6 of Fig. 5;

Fig. 7 is an enlarged schematic perspective view of a plurality of layers of hollow fibres in the oxygenator element of this invention;

Fig. 8 is a view illustrating the spiral wrapping of the tape on a cylindrical core;

Fig. 9 is an enlargement of the oxygenator element illustrating the gap location between contiguous layers of hollow fibres and the blood flow path through the element during oxygenation use.

Fig. 10 is a vertical rear view of the assembly of this invention;

Fig. 11 is a vertical cross-sectional view of the assembly of Fig. 10 showing a blood reservoir secured on top of a blood heater which is secured to the top of the blood oxygenator device of this invention.

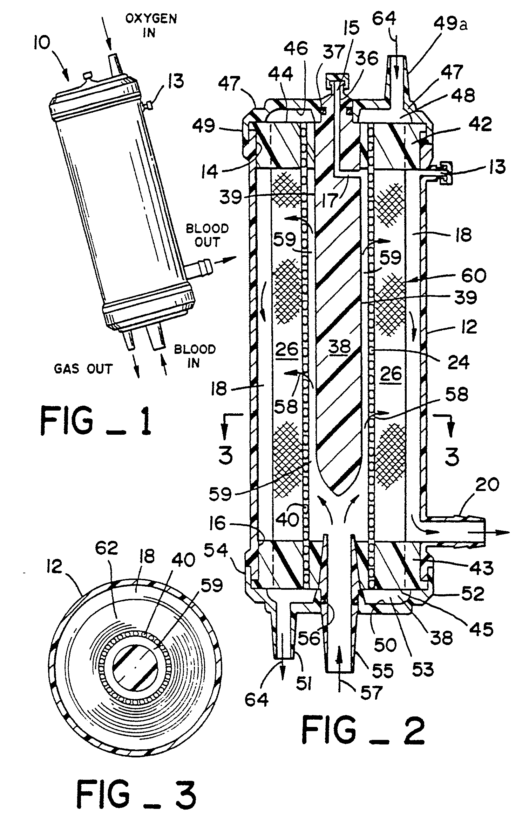

Referring to the drawings the hollow fibre blood oxygenator device 10 comprises a shell 12 and a blood oxygenator element generally designated 60. As best seen in Figs. 2 and 3, oxygenator element 60 consists of an annular mat comprising a plurality of overlying layers of hollow fibres 62 supported on the periphery of blood permeable core member 40. Fibres 62 extend for the full length of core 40 and are secured to each other and to core 40 by an axially spaced resin upper tubesheet 42 and lower tubesheet 43 that seal element 60 to the upper and lower inner wall surfaces 14, 16, respectively of shell 12 adjacent each of its ends to form outer annular blood chamber 18 that is fluid tight.

Tubesheets 42, 43 are generally similar to those employed in hollow fibre separatory elements and devices of the type shown in Mahon U.S. Patent 3,228,867 and may be satisfactorily formed by centrifugal potting techniques describes in Geary et al U.S. Patent 3,442,002 using a suitable resin, for example, polyurethane. Alternatively, tubesheets 42 may be formed by conventional dunk potting techniques now well known to those skilled in the art of hollow fibre devices.

Tubesheets 42, 43 terminate in outer end planar surfaces 44, 45, respectively, and the open ends, or lumens 68 of fibres 62 terminate in the planar face of surfaces 44, 45.

Tubesheet surface 44 forms with the inner wall 46 of upper header 47 an inlet gas chamber 48 that is gas tight and sealed from blood annulus 18 by overlapping flange 49 that is sealed to shell 12 and tubesheet 42, as shown. Tubesheet surface 45 forms with the inner wall 50 of lower header 52 an outlet gas chamber 53 that is gas tight and sealed from blood annulus 18 by flange 54 that is sealed to shell 12 and tubesheet 43, as shown.

Header 47 is provided with inlet gas port 49a which communicates with chamber 48 and during use is attached to a supply gas line, not shown, which normally supplies oxygen, or oxygen containing about 3 to 5 percent carbon dioxide, to the open lumens 68 which lie in the planar surface 44 of tubesheet 42.

Header 52 is provided with outlet gas port 51 that communicates with chamber 53 as shown. It is to be understood, however, that the direction of gas flow, which is preferably downward as shown by arrows 64, may be reversed, if desired. In either event, the gas fed to the inlet chamber enters the lumens of fibres 62 and travels along a spiral path around core 40 and delivers the exit gas to the opposite outlet chamber for discard through means not shown. Header 52 is provided with blood inlet port 55 that is sealed thereto by o-ring 56 and to tubesheet 43, as shown, to deliver blood into blood annulus 59 which lies between the inner surface of core 40 and outer surface 39 of blood flow guide 38. Blood passes through the openings in the wall of core 40 radially outwardly through hollow fibre mat 60 to blood annular space 18 and the oxygenated and purified blood then exits through blood exit port 20. Blood flow through mat 60 will be described in greater detail hereinafter.

Shell 12 is provided with a gas vent 13 located adjacent the upper end of blood annulus 18 that serves as a bubble trap vent for air, or other gas, which may become inadvertently introduced into the blood being processed through mat 60. The blood oxygenator 10 when used in configuration shown in Figs. 1 and 2 is preferably tilted slightly from vertical during use, as shown in Fig. 1 to ensure the bubble vent is the uppermost part of blood annulus 18.

A core vent port 15 communicates through channel 17 in blood guide 38 with the upper end of blood core annulus 59 to permit venting of any air, or other gas bubbles, which may collect during use of oxygenator 10. Blood guide 38 is a pointed cylindrical member sealed into tubesheet 42 and to header 47 by o-ring seal 37 in the upper snout portion 36, as best seen in Fig. 2.

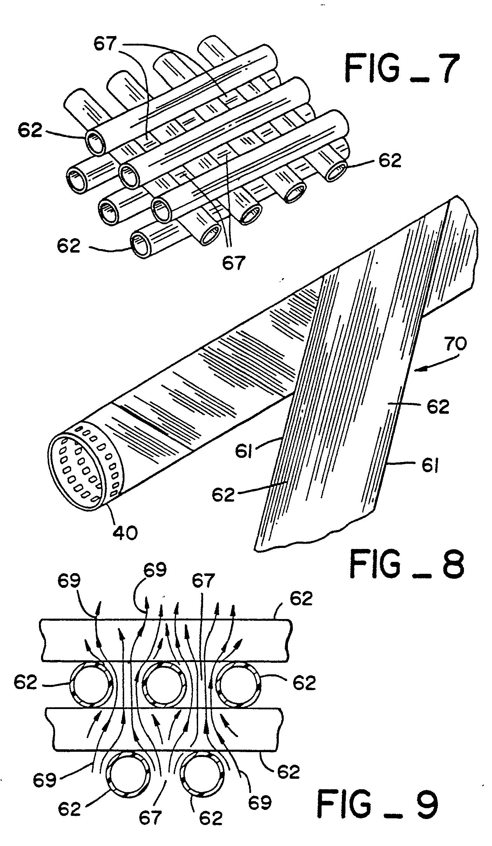

Blood oxygenator element, or mat, 60 is formed, in accordance with the method of this invention, by using a tape, generally designated 70, Fig. 8, of gas permeable hollow fibres 62, that is shown in Figs. 4—8 inclusive. Referring to Figs. 4 and 5, tape 70 consists of a plurality of scores, or hundreds, or thousands of gas permeable hollow fibres arranged in a layer of long, continuous parallel fibres substantially equally spaced from each other and fixed in position by a solid, flexible, small diameter monofilament cross thread 63 which is woven over and under adjacent fibres, Fig. 6, at a selected tension to avoid any substantial crushing or deforming of the fibres at the cross over points 65.

Thread 63 may be any blood compatible material that can be formed into a monofilament that is sufficiently flexible to bend easily over and under the small hollow fibres 62 and which possesses sufficient tensile strength to maintain fibres 62 in their fixed, spaced apart relationship during use. Nylon monofilaments are preferred and satisfactory alternates are polypropylene, impact polystyrene, ABS, or the like. Satisfactory hollow fibres 62 include microporous polyolefins, preferably polyethylene hollow fibres, such as the fibres described in U.S. Patent 4,020,230 having a preferred internal diameter of about 200—400 microns, an outside diameter of about 250 to 500 microns, preferably 300—350 microns, and a spacing between the peripheral edges of adjacent fibres of about 50 to about 300 microns, preferably about 100 microns, monofilament 63 is about 10 to 100 denier, preferably about 15 denier. Cross thread 63 is woven into tape 70 as a continuous thread which overlaps each of the side edge, solid, nonporous monofilaments 61, as shown at 66 in Fig. 5, and preferably range from about 6 to 15 per inch i.e. per 25.4 mm, preferably ubout 12 per inch i.e. per 25.4 mm of length of tape 70.

An open weave tape having a continuously traversing monofilament 61 as described is the preferred form of tape 70, but satisfactory results are also obtained with a non-woven tape in which the parallel hollow fibres are maintained in fixed relationship by a thermoplastic monofilament that forms an adhesive bond with each fibre at the crossover point. Such monofilament can be applied as a molten strand which rigidifies and adheres to the hollow fibres 62 at crossover points 65, and sags slightly between the fibres, and may advantageously have sizes approximately similar to those of the above described nylon monofilaments 63.

Side edge monofilaments 61 are solid, high tensile strength filaments having good abrasion resistance that are selected to have an outside diameter approximately equal to the outside diameter of the hollow fibres in tape 70. Monofilaments 61 successfully withstand the transverse stresses, impact and abrasion wear at turns of the shuttle during weaving and also serve to relieve the longitudinal tensioning forces that are required to maintian fibres 62 in their spaced apart relationship.

Nylon is the preferred material for side edge monofilaments 61 and may vary between about 200 to about 500 microns to correlate with the selected hollow fibres. As an example, in a tape 70 having 160 hollow fibres of microporous polyethylene, each fibre having an outside diameter of about 300 microns, a solid nylon monofilament of 250 micron diameter was used satisfactorily.

Tape 70 may satisfactorily contain a wide range of numbers of hollow fibres that will vary somewhat with hollow fibre size. The number is selected so as to form a tape having a width that is complementary to the outside diameter of core 40 to enable satisfactory spiral winding to form annular mat 60 by using the technique illustrated in Fig. 8. For example, with 325 micron outside diameter microporous polyethylene hollow fibres having a spacing of 100 microns between fibres, an easily handleable tape 70 having a width of about 2.6 inches i.e. 66.04 mm was formed by using 160 hollow fibres. By using such tape, and a core 40 having a 1.25 inch (i.e. 31.75 mm) outside diameter, tape is applied to the core, as the core rotates, by controlling the angle of divergence of the tape from the longitudinal axis of the core such that the side edge filaments 61 lie in immediate adjacency continuously, as successive widths of tape 70 cover the peripheral surface of core 40 for the entire length of the core, before reversal of the angle of the tape to traverse the length of the core in the opposite direction. It is also satisfactory to position two or more tapes 70 in side by side relationship for simultaneous application to the periphery of core 40 for each axial rotation of the core.

It has been found that the optimum annular mat 60 is one which provides contiguous individual fibres in overlying layers of fibres that intersect at an angle in the range of about 45 to 90° and preferably close to 90° and not less than about 30°. As best seen in Fig. 7, gaps 67 are substantially square in shape for fibres 62 in contiguous layers which intersect at an angle of substantially 90° and the gap shape becomes an increasingly flattened parallelogram shape as the angle of crossover decreases toward 45°. Fig. 9 illustrates the path of blood flow over the fibres 62 and through gaps 67 as it moves radially along the paths 69 from core annulus 59 to arterial blood annulus 18.

Blood oxygenator element 60 may advantageously be surrounded with a layer of blood filtering screening material such a nylon screen having pores somewhat smaller than the average lateral spacing between hollow fibres in the mat layers, for example, about 20 to 60 microns.

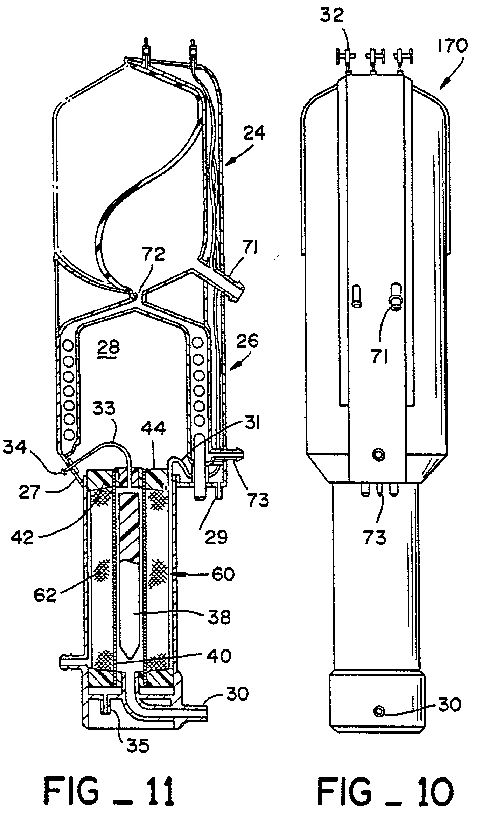

As seen in Figs. 10 and 11 the blood oxygenator element 60 is incorporated into a unitary assembly comprising in vertical array a top blood reservoir generally designated 24, attached at its lower end to the top of a blood heater, generally designated 26, and having blood oxygenator element 60 secured to the lower end of heater 26. As shown in Fig. 11, the shell 12 has been modified to enable attachment of the upper wall portions of shell 12 to the lower end of housing 27 of heater 26. Header 47 has been removed so as to expose the upper surface 44 of tubesheet 42 to the interior core portion of heater 26 which becomes a gas chamber 28 that receives oxgyen through inlet 29 which passes downwardly through hollow fibres 62 and exits through outlet port 35. The shape of blood inlet 55 of the oxygenator device shown in Fig. 2 is modified to exit at a location on the rear surface of the assembly as shown at 30. Blood entering at inlet 30 passes upwardly through blood annular chamber 59, through walls of core 40, through element 60 into blood annulus 18 and the purified arterial blood passes outwardly through port 20 and returns to the patient.

Bubble vents 13 and 15 as shown in Fig. 2 have been modified as to location by adding connecting tube 31 to vent port 32, and the tube 33 to vent port 34, respectively.

Blood heater 26 and blood reservoir 24 may be of conventional construction, or as shown, blood reservoir 24 may have the shape and construction which is described in detail in our co-pending Application No. 83 300 873.3 (published as EP—A—0 089 748 on 28.09.83).

During use of the assembly 170, Fig. 10, venous blood enters reservoir 24 at inlet 71, passes downwardly through blood outlet 72 into heater 26 and blood, heated or cooled to the desired temperature, exits from blood outlet 73 and is delivered to blood inlet 30 by suitable means, not shown.

**Claims**

1. A blood oxygenator element (60) for the treatment of blood comprising a plurality of hollow gas permeable fibres (62) and a longitudinal hollow blood permeable core (40), said fibres (62) overlying the peripheral surface of said core (40) in a plurality of contiguous layers, each layer comprising a multitude of said fibres (62) and extending spirally around and along the longitudinal axis of said core (40), said fibres (62) being spaced laterally from each adjacent fibre in said layer, and said fibres (62) terminating in spaced apart tubesheets (42, 43) joining said fibres to each other and to said core, characterised in that said multitude of fibres in each layer are arranged in a tape (70) with the fibres in each tape extending longitudinally thereof in parallel arrangement distributed across its width, and in that the tapes (70) in alternate layers extend spirally in opposite directions so that the said fibres (62) in each said layer are substantially equally radially distant from said peripheral surface of said core, and that said fibres in each said layer cross the fibres (62) in each radially adjacent layer at an angle of between 30° and 90°.

2. A blood oxygenator element (60) as claimed in claim 1, characterised by a solid monofilament (61) defining each of the side edges of each tape (70), and a solid flexible transverse filament (63) for maintaining spacing between said hollow fibres (62) without substantially altering their cross-section at crossover points (65) therewith, said solid flexible transverse filament (63) being woven over and under the adjacent parallel hollow fibres (62) and around the exterior of said monofilament side edges (61) in a continuous traversing pattern along the length of said tape (70).

3. A blood oxygenator element (60) as claimed in any one of claims 1 or 2, characterised in that the exterior surfaces of the fibres in each said layer contact the exterior surfaces of the adjacent fibres in each radially contiguous layer.

4. A blood oxygenator element (60) as claimed in any one of claims 1 to 3, characterised in that the tape in each layer extends spirally around said core in multiple spirals, and one edge of the tape in each layer lies closely in side-by-side arrangement with the opposite edge of that tape in the proceeding spiral of the same layer.

5. A blood oxygenator element (60) as claimed in any one of claims 1 to 4, characterised in that said core is cylindrical, and each said layer extends the full length of said core.

6. A blood oxygenator element (60) as claimed in any one of claims 1 to 5, characterised in that said fibre is polypropylene.

7. A blood oxygenator element (60) as claimed in any one of claims 1 to 5, characterised in that said gas permeable fibres have an oxygen permeability of at least $2 \times 10^{-5}$ cc (STP) per cm$^2$ of membrane area per second per cm Hg transmembrane pressure differential.

8. A blood oxygenator element (60) as claimed in claim 7, characterised in that said core is cylindrical, and said gas permeable fibres consist essentially of polyethylene.

9. A blood oxygenator (10) comprising a tubular shell (12) forming a liquid tight annular chamber (18) around a blood oxygenator element (60), a gas tight chamber (48, 53) adjacent to outer end surfaces of each of tubesheets (42, 43) of said element, port means (49a, 51) communicating with each gas chamber (48, 53), blood inlet means (55) extending through one of said tubesheets (43) and communicating with core (40) of said element, and blood outlet means (20) communicating with said liquid tight annular chamber (18), characterised in that said blood oxygenator element (60) is as claimed in any one of claims 1 to 8.

10. A blood oxygenator as claimed in claim 9, characterised in that it also comprises bubble vent means (13, 15) communicating with the upper end portions of said liquid tight annular chamber (18) around said element and the interior (59) of said core (40).

11. An assembly comprising a blood oxygenator as claimed in claim 9 or 10, characterised in that it also comprises a self-supporting unit having an upper blood reservoir (24) attached at its lower end to the upper end of a blood heater (26), said blood heater (26) being attached at its lower end to the upper end of said shell (12).

12. A blood oxygenator element (60) as claimed in claims 1 to 8, characterised in that said tape is an open weave tape having a solid monofilament (61) defining each of the side edges of said tape (70) and a solid flexible transverse filament (63) woven over and under said adjacent parallel hollow fibres (62) and around the exterior of said solid side edge monofilament (61) in a continuous traversing pattern along the length of said tape; said transverse filament (63) maintaining spacing between said hollow fibres (62) without substantially altering the circular cross section of said hollow fibres (62) at crossover points (65) therewith.

13. A method for making a blood oxygenator element as claimed in claim 1, which method comprises:

1. providing a core (40),
2. providing a tape (70) of hollow gas permeable fibres (62), said tape comprising a plurality of said fibres arranged in substantially parallel spaced apart and substantially planar relation,
3. spirally wrapping said tape (70) on said core to form a plurality of layers of said fibres overlying the peripheral surface of said core, the width of said tape and the diameter of said core being selected such that the edge surface of each successive wrap of said tape in said layer is adjacent to the edge surface of the prior wrap so as to substantially maintain said substantially parallel spaced apart relation between said fibres in each said tape, and covering the peripheral surface of said core for the length of said core, and
4. securing the axially spaced apart ends of said fibres in each layer to each other and to said core in tubesheets (42, 43) having coplanar outer end surfaces (44, 45) in which the open lumens (68) of each said fibre (62) terminate, characterised in that
5. said wrapping to form overlying layers covering the prior formed said layer is achieved by reversing the direction of said spiral such that each tape wrap positions each said fibre in said overlying layer relative to the adjacent fibre in the underlying layer at an angle between about 30° and about 90°, and
6. said core is a hollow blood permeable core.

14. A method as claimed in claim 13, characterised in that said wrapping step includes application of a plurality of said tapes for each rotation of said core.

**Patentansprüche**

1. Blutsauerstoff erzeugendes Element (60) zur Behandlung von Blut mit einer Vielzahl von hohlen gasdurchlässigen Fasern (62) und einem longitudinalen hohlen blutdurchlässigen Kern (40), wobei die Fasern (62) auf der Umfangsflache des Kerns (40) in einer Vielzahl einander benachbarter Schichten abgelagert sind und jede Schicht eine Anzahl von Fasern (62) enthält, die sich spiralförmig um die Längsachse des Kerns (40) erstrecken, wobei die Fasern (62) in Querrichtung einen Abstand von jeder benachbarten Faser in der Schicht aufweisen und die Fasern (62) in Kopfplatten (42, 43) enden, die einen Abstand voneinander aufweisen und die Fasern jeweils miteinander und mit dem Kern verbinden, dadurch gekennzeichnet, daß die Menge der Fasern in jeder Schicht in Form eines Bandes (70) angeordnet ist, wobei sich die Fasern jedes Bandes (70) in Längsrichtung desselben parallel zueinander verteilt über die Breite des Bandes erstrecken, so daß die Fasern (62) in jeder Schicht im wesentlichen gleichen radialen Abstand von der Außenoberfläche des Kerns haben und daß die Fasern in jeder Schicht die Fasern (62) in jeder radial benachbarten Schicht unter einem Winkel zwischen 30° und 90° kreuzen.

2. Blutsauerstoff erzeugendes Element (60) nach Anspruch 1, gekennzeichnet durch ein festes Monofilament (61), das die Seitenkante jedes Bandes (70) bildet und ein festes flexibles quer hin und her verlaufendes Filament (63) zum Aufrechterhalten des Abstandes zwischen den hohlen Fasern (62), ohne ihren Querschnitt wesentlich an den Kreuzungspunkten (65) mit dem Filament zu verandern, wobei das feste flexible quer hin und der verlaufende Filament (63) über und unter benachbarte parallele Hohlfasern (62) und um die Außenseite der Seitenkantenfilamente (61) gewebt ist, in einem kontinuierlich kreuzenden Muster über die Länge des Bandes (70).

3. Blutsauerstoff erzeugendes Element (60) nach jedem der Ansprüche 1 bis 3, dadurch gekennzeichnet, daß sich die Außenoberflachen der Fasern jeder Schicht und die Außenoberflächen der benachbarten Fasern in jeder radial benachbarten Schicht berühren.

4. Blutsauerstoff erzeugendes Element (60) nach jedem der Ansprüche 1 bis 3, dadurch gekennzeichnet, daß das Band sich in jeder Schicht spiralförmig um den Kern in vielen Spiralen erstreckt und eine Kante des Bandes in jeder Schicht dicht Seite an Seite mit der gegenüberliegenden Kante des Bandes der fortschreitenden Spirale in der gleichen Schicht liegt.

5. Blutsauerstoff erzeugendes Element (60) nach jedem der Ansprüche 1 bis 4, dadurch gekennzeichnet, daß der Kern zylindrisch ist und sich jede Schicht über die volle Lange des Kerns erstreckt.

6. Blutsauerstoff erzeugendes Element nach jedem der Ansprüche 1 bis 5, dadurch gekennzeichnet, daß die Faser Polypropylen ist.

7. Blutsauerstoff erzeugendes Element (60) nach jedem der Ansprüche 1 bis 5, dadurch gekennzeichnet, daß die gasdurchlassigen Fasern eine Sauerstoffdurchlässigkeit von mindestens $2 \times 10^{-5}$ cc (STP) pro cm$^2$ Membranflache pro Sekunde pro cm Hg Druckabfall über die Membrane aufweisen.

8. Blutsauerstoff erzeugendes Element (60) nach Anspruch 7, dadurch gekennzeichnet, daß der Kern zylindrisch ist und die gasdurchlässigen Fasern im wesentlichen aus Polyethylen bestehen.

9. Blutsauerstofferzeuger (10) mit einer Außenwand (12), die einen flussigkeitsdichten Ringraum (18) um ein Blutsauerstoff erzeugendes Element (60) bildet, einer gasdichten Kammer (48, 53) benachbart den äußeren Endflachen jeder Kopfplatte (42, 43) des Elementes, Anschlußstutzen (49a, 51) in Verbindung mit jeder Kammer (48, 53), Bluteinlaßeinrichtungen (55), die sich durch eine der Kopfplatten (43) erstrecken und mit dem Kern (40) des Elementes verbunden sind und Blutauslaßeinrichtungen (20), die mit dem flüssigkeitsdichten Ringraum (18) verbunden sind, dadurch gekennzeichnet, daß das Blutsauerstoff erzeugende Element ein Element nach jedem der Ansprüche 1 bis 8 ist.

10. Blutsauerstofferzeuger nach Anspruch 9, dadurch gekennzeichnet, daß er außerdem Gasentluftungsventile (13, 15) enthält, die mit den oberen Endteilen des flüssigkeitsdichten Ringraumes (18) um das Element und dem Inneren (59) des Kerns (40) verbunden sind.

11. Baugruppe enthaltend einen Blutsauerstofferzeuger nach Anspruch 9 oder 10, dadurch gekennzeichnet, daß die außerdem enthält eine selbsttragende Einheit mit einem oberen Blutbehalter (24), dessen unteres Ende mit dem oberen Ende eines Blutheizers (26) verbunden ist und das untere Ende des Blutheizers (26) mit dem oberen Ende der Außenwand (12) verbunden ist.

12. Blutsauerstoff erzeugendes Element (60) nach jedem der Ansprüche 1 bis 8, dadurch gekennzeichnet, daß das Band ein offenes gewebtes Band ist mit einem festen Monofilament (61), das jeweils die Seitenkanten des Bandes (70) bildet und einem festen flexiblen quer hin und her verlaufenden Filament (63), das über und unter die benachbarten parallelen Hohlfasern (62) und um das Äußere des festen Seitenkanten-Monofilamentes (61) in einem endlösen kreuzenden Muster über die Lange des Bandes gewebt ist, wobei das quer hin und her verlaufende Filament (63) die Abstande zwischen den Hohlfasern (62) aufrechterheit, ohne den kreisförmigen Querschnitt der Hohlfasern (62) an den Kreuzungspunkten (65) mit dem Filament wesentlich zu verändern.

13. Verfahren zum Herstellen eines Blutsauerstoff erzeugenden Elementes nach Anspruch 1, durch

1) Schaffen eines Kerns (40),

2) Herstellen eines Bandes (70) aus gasdurchlässigen Hohlfasern (62), wobei das Band eine Vielzahl von Fasern im wesentlichen parallel in Abstand voneinander und im wesentlichen in einer Ebene aufweist,

3) spiralformiges Aufwickeln des Bandes (70) auf den Kern, um eine Vielzahl von Schichten der Fasern auf der Umfangsfläche des Kerns auszubilden, wobei die Breite des Bandes und der Durchmesser des Kerns so ausgewählt sind, daß sich die Kantenoberfläche jeder aufeinanderfolgenden Windung des Bandes in der Schicht benachbart der Kantenoberfläche der vorherigen Windung befindet, um im wesentlichen den parallelen Abstand zwischen den Fasern in jedem Band aufrechtzuerhalten und Bedecken der Umfangsfläche des Kerns über die Länge des Kerns und

4) Befestigen der in axialem Abstand voneinander befindlichen Enden der Fasern jeder Schicht miteinander und mit dem Kern durch Kopfplatten (42, 43), die in der gleichen Ebene liegenden Endflächen (44, 45) aufweisen, in denen die offenen Lumen (68) jeder der Fasern (62) enden, dadurch gekennzeichnet, daß

5) beim Wickeln zum Ausbilden von übereinanderliegenden Schichten, die die jeweils zuvor gebildete Schicht bedecken, die Richtung der Spirale umgekehrt wird, so daß jede Bandwickellage jeder Faser in der darüberliegenden Schicht zur benachbarten Faser in der darunterliegenden Schicht unter einem Winkel zwischen etwa 30 und etwa 90° verlauft und

6) der Kern ein hohler, blutdurchlässiger Kern ist.

14. Verfahren nach Anspruch 13, dadurch gekennzeichnet, daß der Wickelschritt das Aufbringen einer Vielzahl von Bändern bei jeder Drehung des Kerns einschließt.

**Revendications**

1. Element (60) d'oxygenation de sang pour le traitement du sang, comprenant plusieurs fibres (62) creuses permeables au gaz et un noyau (40) creux longitudinal permeable au sang, lesdites fibres (62) étant disposees sur la surface peripherique dudit noyau (40) en plusieurs couches contigués, chaque couche comprenant une multitude desdites fibres (62) et s'étendant en spirale autour de l'axe longitudinal dudit noyau (40) et le long de cet axe, lesdites fibres (62) etant espacees lateralement de chaque fibre adjacente dans ladite couche, et lesdites fibres (62) se terminant par des plaques tubulaires (42, 43) espaces qui relient lesdites fibres les unes aux autres et audit noyau, caracterise en ce que ladite multitude de fibres dans chaque couche est disposée en une bande (70), les fibres dans chaque bande s'étendant dans le sens longitudinal de celle-ci dans une configuration parallele repartie sur la largeur de la bande, et que les bandes (70) s'etendant dans des couches alternantes s'etendant un spirale dans des sens opposes, de sorte que lesdites fibres (62) dans chaque couche se trouvent a une distance radiale sensiblement egale de la surface peripherique dudit noyau, et que lesdites fibres dans chacune desdites couches croisent les fibres (62) dans chaque couche radialement adjacente, sous un angle situe entre 30° et 90°.

2. Element (60) d'oxygenation de sang selon la revendication 1, caracterise par un monofilament (61) plein delimitant chacun des bords lateraux de chaque bande (70), et par un filament (63) plein transversal souple destiné à maintenir l'espacement entre lesdites fibres creuses (62) sans modifier sensiblement leur section aux points de croisement (65) avec celles-ci, ledit filament (63) plein transversal souple etant tisse au-dessus et au-dessous des fibres (62) creuses paralleles adjacentes et autour de l'exterieur desdits bords latéraux (61) en monofilament, en un dessin transversal continu le long de ladite bande (70).

3. Element (60) d'oxygenation de sang selon la revendication 1 ou 2, caractérisé en ce que les surfaces exterieures des fibres dans chacune desdites couches sont en contact avec les surfaces exterieures des fibres adjacentes dans chaque couche radialement contigue.

4. Element (60) d'oxygenation de sang selon l'une quelconque des revendications 1 à 3, caracterise en ce que la bande dans chaque couche s'etend en plusieurs spirales autour dudit noyau, et qu'un bord de la bande dans chaque couche se trouve dans une disposition cote-à-côte etroite avec le bord oppose de cette bande dans la spirale precedente de la meme couche.

5. Element (60) d'oxygenation de sang selon l'une quelconque des revendications 1 à 4, caracterise en ce que ledit noyau est cylindrique, et que chacune desdites couches s'etend sur toute la longueur dudit noyau.

6. Element (60) d'oxygenation de sang sleon l'une quelconque des revendications 1 à 5, caracterise en ce que ladite fibre est en polypropylene.

7. Element (60) d'oxygenation de sang selon l'une quelconque des revendications 1 à 5, caracterise en ce que lesdites fibres permeables au gaz possedant une permeabilité à l'oxygene d'au moins $2 \times 10^{-5}$ cc (STP) par cm$^2$ de superficie de membrane par seconde par cm Hg de differentiel de pression transmembrane.

8. Element (60) d'oxygenation de sang selon la revendication 7, caracterise en ce que ledit noyau est cylindrique et que lesdites fibres permeables au gaz sont essentiellement en polyethylene.

9. Oxygenateur de sang (10) comprenant une enveloppe tubulaire (12) formant une chambre annulaire (18) etanche aux liquides autour d'un élement (60) d'oxygenation de ssng, une chambre (48, 53) etanche aux gaz adjacente aux surfaces d'extremité exterieure de chacune des plaques tubulaires (42, 43) dudit element, un moyen de passage (49a, 51) communiquant avec chaque chambre (48, 53) etanche au gaz, un moyen (55) d'admission de sang s'etendant a travers une desdites plaques tubulaires (43) et communiquant avec le noyau (40) dudit élement, et un moyen (20) de sortie de sang communiquant avec ladite chambre annulaire (18) étanche aux liquides, caractérisé en ce que ledit élement d'oxygenation de sang (60) correspond à celui de l'une quelconque des revendications 1 à 8.

10. Oxygenateur de sang (10) selon la revendication 9, caractérisé en ce qu'il comprend également un orifice (13, 15), d'échappement de bulles vers l'atmosphère, communiquant avec les portions d'extremite superieures de ladite chambre annulaire (18) étanche aux liquides autour dudit élement et de l'interieur (59) dudit noyau (40).

11. Assemblage comprenant un oxygenateur de sang selon la revendication 9 ou 10, caractérisé en ce qu'il comprend également un ensemble autoportant comportant un reservoir de sang (24) superieur fixe par son extremite inférieure à l'extremite superieure d'un élément (26) de chauffage de sang, ledit élément (26) de chauffage de sang étant fixe par son extrémite inférieure à l'extrémite supérieure ladite enveloppe (12).

12. Element d'oxygenation de sang (60) selon l'une quelconque des revendications 1 à 8, caractérisé en ce que ladite bande est une bande tissee ouverte qui possede un monofilament (61) plein délimitant chacun des bords lateraux de ladite bande (70) et un filament (63) plein transversal souple tisse au-dessus et au-dessous desdites fibres (62) creuses paralleles adjacentes et autour de l'extérieur dudit monofilament plein (61) de bord lateral, en un dessin transversal continu le long de ladite bande: ledit filament (63) transversal maintenant l'éspacement entre lesdites fibres creuses (62) sans modifier sensiblement la section circulaire desdites fibres creuses (62) aux points de croisement (65) avec celles-ci.

13. Methode pour la fabrication d'un élément d'oxygenation de sang selon la revendication 1, methode comprenant:

1) le recours a un noyau (40).

2) le recours à une bande (70) de fibres (62) creuses permeables au gaz, ladite bande comprenant plusieurs desdites fibres disposees dans une relation sensiblement paralléle espacee et sensiblement plane.

3) l'enroulement en spirale de ladite bande (70) sur ledit noyau pour former plusieurs couches desdites fibres recouvrant la surface periphérique dudit noyau, la largeur de ladite bande et le diamétre dudit noyau étant sélectionnes de telle maniere que la surface de bord de chaque enroulement successif de ladite bande dans ladite couche est adjacente à la surface de bord de l'enroulement précédent, de manière à maintenir sensiblement ladite relation sensiblement parallele espacee entre lesdites fibres dans chaque bande, et la recouvrement de la surface périphérique dudit noyau sur toute la longueur de celui-ci, et

4) la fixation des extrémites axialement espacées desdites fibres dans chaque couche les unes aux autres et audit noyau dans des plaques tubulaires (42, 43) possédant des surfaces coplanaires (44, 45) d'extremité, dans lesquelles se terminent les canaux (68) ouverts de chacune desdites fibres (62), caractérisée en ce que

5) ledit encoulement destiné à former des couches superposees recouvrant les couches formées précédemment est achevé en inversant la direction de ladite spirale de telle façon que chaque enroulement de bande positionné chacune desdites fibres dans ladite couche superposée, par rapport à la fibre adjacente dans la couche sousjacente, sous un angle situe entre environ 30° et environ 90°, et que

6) ledit noyau est un noyau creux permeable au sang.

14. Methode selon la revendication 13, caractérisée en ce que l'étape d'enroulement comprend l'application de plusieurs desdites bandes pour chaque rotation dudit noyau.

FIG _ 1

FIG _ 2

FIG _ 3

FIG _ 4

62
62
61
61
63
62

65
6
66
62
68
6
62
63
68
FIG _ 5
61
68

65   63   65
61
62   68   65   68   62   61
FIG _ 6

FIG _ 7

FIG _ 8

FIG _ 9

FIG _ 11

FIG _ 10